Europäisches Patentamt

European Patent Office

Office européen des brevets

(19)

(11) Publication number: **0 137 657 B1**

# EUROPEAN PATENT SPECIFICATION

(12)

(45) Date of publication of patent specification: **17.04.91**

(51) Int. Cl.⁵: **C12P 21/08, C12N 5/00, G01N 33/535**

(21) Application number: **84305570.8**

(22) Date of filing: **16.08.84**

(54) Monoclonal antibodies.

(30) Priority: **16.08.83 ZA 836016**

(43) Date of publication of application:
**17.04.85 Bulletin 85/16**

(45) Publication of the grant of the patent:
**17.04.91 Bulletin 91/16**

(84) Designated Contracting States:
**AT BE CH DE FR GB IT LI LU NL SE**

(56) References cited:
**EP-A- 0 068 763**
**DD-A- 228 824**
**US-A- 3 773 625**

**BIOLOGICAL ABSTRACTS, vol. 77, no. 9, 1984, abstract no. 67644; J.P. VAN DER WEIJ et al.: "The use of a peroxidase-antiperoxidase complex for the visualization of monoclonal antibodies on the ultrastructural level", & CLIN. EXP. IMMUNOL. 54(3): 819-826, 1983**

(73) Proprietor: **BIOCLONES (PROPRIETARY) LIMITED**
**142 Medical Mews**
**Sandton City Sandton Transvaal(ZA)**

(72) Inventor: **Keogh, Hillary Joan**
**87, St. Andrews Street**
**Hurlingham Sandton Transvaal(ZA)**
Inventor: **Shearer, Richard Gordon**
**146, D.F. Malan Drive**
**Roosevelt Park Johannesburg Transvaal(ZA)**

(74) Representative: **Hildyard, Edward Martin et al**
**Frank B. Dehn & Co. Imperial House 15-19 Kingsway**
**London WC2B 6UZ(GB)**

Rank Xerox (UK) Business Services

NATURE, vol. 305, 6th October 1983, pages 537-540, Macmillan Journals Ltd.; C. MILSTEIN et al.: "Hybrid hybridomas and their use in immunohistochemistry"

GENETIC ENGINEERING, vol. 2, 1980, pages 31-46; S.-P. KWAN et al.: "Production of monoclonal antibodies"

CHEMICAL ABSTRACTS, vol. 100, no. 9, 27th February 1984, page 441, abstract no. 66273f, Columbus, Ohio, US; K. OGATA et al.: "Detection of Toxoplasma membrane antigens transferred from SDS-polyacrylamide gel to nitrocellulose with monoclonal antibody and avidin-biotin, peroxidase antiperoxidase and immunoperoxidase methods"

CHEMICAL ABSTRACTS, vol. 97, no. 17, 25th October 1982, page 503, abstract no. 142804s, Columbus, Ohio, US; D.Y. MASON et al.: "Preparation of peroxidase-antiperoxidase (PAP) complexes for immunohistological labeling of monoclonal antibodies"

J. UOEH 4(2), 1982, pp. 133-138

JOURNAL OF IMMUNOLOGICAL METHOD 58 (1983), pp. 109-118

**Description**

This invention relates to mouse monoclonal antibodies capable of forming complexes with horse radish peroxidase, herein designated monoclonal peroxidase anti-peroxidase (PAP) complexes, and to the hybrid cell lines (hybridomas) which may be used in the preparation of such monoclonal antibodies.

The monoclonal antibodies of the invention, the PAP complexes derived from them and compositions containing PAP complexes are useful in research and diagnosis including immunohistological methods.

Whilst the fusion of mouse myeloma cells to spleen cells from immunised mice to produce hybridomas which secrete monoclonal antibodies is known in the art, (Kohler and Milstein, Nature, 256, 495-497) there are many difficulties to be overcome and variations required for each specific case. For any given case, the choice of animal species, and myeloma cell line and the selection technique used for isolating the desired hybridoma cell line are all critical to the outcome. Reference can be had in this regard to, for example, Current Topics in Microbiology and Immunology, Volume 81, F. Melchers, M. Patter, N. Warner, Springer-Verlag, 1978 and Handbook of Experimental Immunology, Third Edition, Volume 2, chapter 25, D.M. Wier, Editor, Blackwell, 1978.

There is no assurance prior to attempting to prepare a hybridoma producing a desired monoclonal antibody that one will be successful, that the hybridoma will be stable enough to produce the antibody under practical conditions of production, if at all, and that the antibody will have the desired properties.

References which describe methods of producing peroxidase-containing complexes and monoclonal antibodies against horse radish peroxidase are:

1. Mason D.Y., Cordell, J., Abdulaziz, Z., Naiem, M. and Bordenave G., 1982. Preparation of Peroxidase : Antiperoxidase Complexes for Immunohistological Labelling of Monoclonal Antibodies. Journal of Histochemistry and Cytochemistry 30, (11) 1114-1122, describes the preparation of a monoclonal antibody against horse radish peroxidase using Balb/c mice and the myeloma cell line NS1 (presumably P3-NS1/Ag4-1). This reference also describes the preparation of a PAP complex using the antibody. No characterisation of the complex is provided except the molecular weight which is stated to be 340,000. These complexes cannot be the same as those forming the subject of the present invention.

2. Okai, Y., Yamaguchi, Y. and Nakamura, H., 1982. Production of Hybridoma Secreting Specific Antibody against Horse Radish Peroxidase Isoenzyme. J UOEH, 4 (2) 133-138, describes the use of Balb/c mice and the myeloma cell line P3-X63-U1 AZG.

3. Ternynck, T., Gregoire, J. and Aurameas, S., 1983. Enzyme/Antienzyme Monoclonal Antibody Soluble Complexes (EMAC) : Their use in Quantitave Immunoenzymatic Assays. Journal of Immunological Methods, (58) 109-118, describes the use of C57BL/6 mice and Sp2-O cells.

SUMMARY OF THE INVENTION

According to one aspect of the invention, there is provided a mouse monoclonal anti-peroxidase antibody characterised in that it is:

(a) of the sub-class $IgG_1$;

(b) capable of binding with horse radish peroxidase to form a peroxidase anti-peroxidase complex without reducing the catalytic activity of the enzyme by more than 30% the complex having a molar ratio of peroxidase to antibody of at least 1.78:1, (preferably 1,96:1) and having an RZ value of at least 0,55, (preferably at least 0,59).

The antibody is preferably produced by a hybridoma formed by fusion of spleen cells from a Balb/c mouse previously immunised with horse radish peroxidase and cells from the mouse myeloma cell line Sp2/O-Ag14. The hybridoma is preferably that which, when injected into the peritoneal cavity of a pristane treated Balb/c mouse produces an average of at least 5,5ml, preferably at least 9,5ml, of ascites fluid on 3 taps, at 2 day intervals between taps.

According to another aspect of the invention, there is provided a hybridoma formed by fusion of spleen cells from a Balb/c mouse previously immunised with horse radish peroxidase and cells from an Sp2/O-Ag14 mouse myeloma cell line capable of producing a monoclonal antibody as described above.

According to yet another aspect of the invention, there is provided a method for preparing a hybridoma, as described above, which comprises the steps of:

(i) immunising a Balb/c mouse, with horse radish peroxidase enzyme;

(ii) removing the spleen from the mouse and extracting spleen cells, for example by making a suspension of the spleen cells;

(iii) fusing the spleen cells with Sp2/O-Ag14 mouse myeloma cells in the presence of a cell fusion

3

promoter;

(iv) diluting and culturing the fused cells in suitable containers in a medium which will not support the unfused myeloma cells preferably in the presence of feeder spleen cells;

(v) examining the supernatant in each container containing hybridomas for the presence of the desired antibody; and

(vi) selecting and cloning, preferably by the technique of limiting dilution, hybridomas producing the desired antibody;

The preferred method of producing such hybridomas comprises the steps of:

(i) immunising a Balb/c mouse with horse radish peroxidase by injecting subcutaneously 10 - 2000 μg of the enzyme in an adjuvant medium, e.g. 0.2 ml, preferably at two sites, followed by subsequent injections at spaced intervals, e.g. then injection at about four-weekly intervals, each of 10 - 2000 μg of the enzyme in an adjuvant medium (e.g 0.2 ml) preferably at one site, followed by a final booster dose of 10-500 μg of the enzyme in saline (e.g. 0.2 ml) administered subcutaneously, preferably at two sites;

(ii) removing the spleen aseptically from the mouse and making a suspension of spleen cells:

(iii) fusing the spleen cells with Sp2/O Ag14 mouse myeloma cells in the presence of a cell fusion promoter, such as polyethylene glycol,

(iv) diluting and culturing the fused cells in suitable containers in a medium which will not support the growth of unfused myeloma cells, for example, a medium containing an appropriate concentration of hypoxanthine, aminopterin and thymidine, in the presence of feeder spleen cells prepared from the spleen of a young mouse;

(v) examining the supernatant in each container containing hybridomas for the presence of desired antibody using an appropriate method such as an enzyme immunoassay (ELISA assay) using β –galactosidase linked antimouse immunoglobulin, produced in a suitable species such as rabbit, sheep, horse; and

(vi) selecting and cloning. preferably by the technique of limiting dilution hybridomas producing the desired antibody.

The hybridomas produced as described above may be placed in a suitable medium to produce antibodies and the antibodies recovered therefrom. Preferably the hybridomas are administered intraperitoneally to Balb/c mice previously treated with an irritant such as pristane, the resultant ascites harvested and the antibody recovered therefrom.

The antibodies described above are useful in producing monoclonal peroxidase anti-peroxidase complexes useful in immunohistological procedures. These complexes form another aspect of the invention.

Preferred methods for putting our invention into effect will now be described by way of illustration only. The invention is further illustrated by reference to the accompanying drawings, wherein;

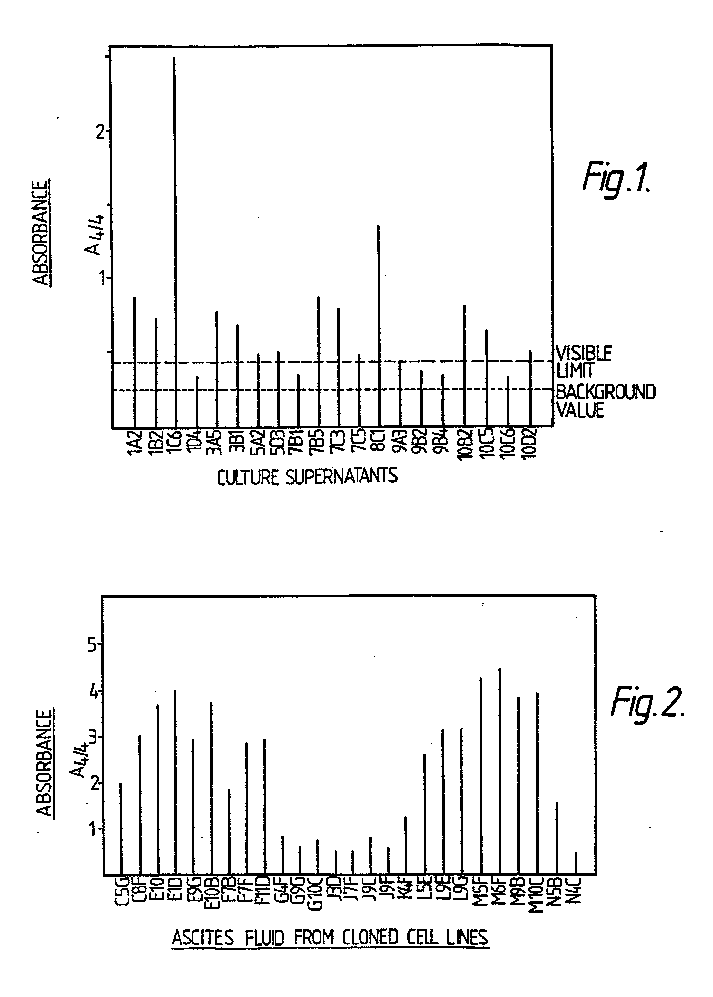

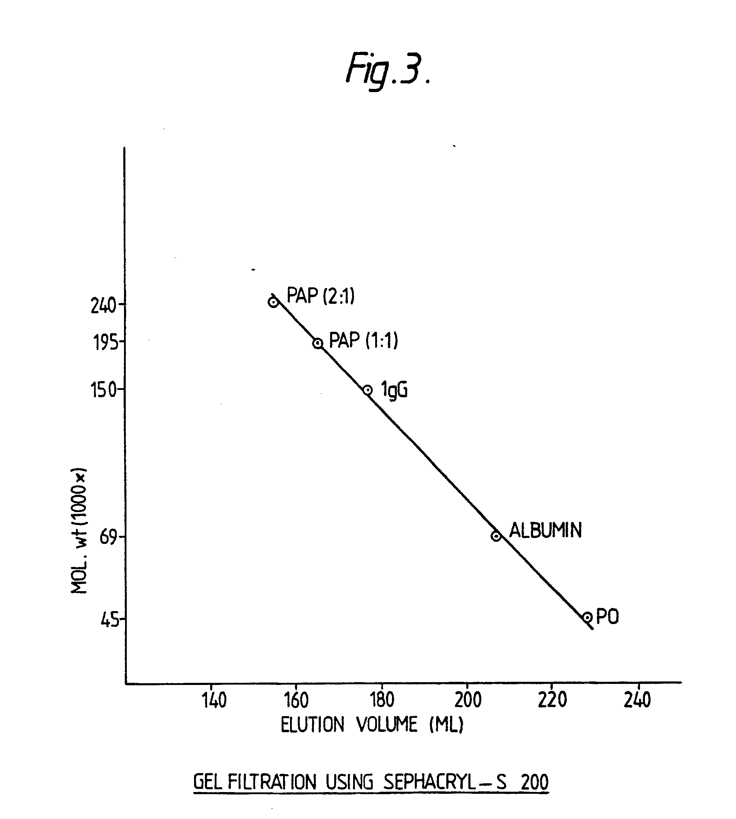

Figures 1 and 2 are plots obtained from subjecting certain supernatants and ascites fluids to the ELISA assay; and Figure 3 is a plot of elution volume against the log of molecular weight in a gel chromatography analysis.

Immunisation, Cell Fusion and Cloning

1.1. Immunisation Procedure

Eight 6-week-old ♀♀ Balb/c mice (source South African Institute for Medical Research) were immunised with horse radish peroxidase (HP) (Bayer Miles 36-451-2, lot 8012 and 8042, RZ = 3,0) as follows:

1st injection : 125 μg HP in 50% saline and 50% Freunds Complete adjuvant (Merck 06-38-60-7) was given in 0.1 ml each at two sites per mouse, subcutaneously.

3 subsequent injections at four-weekly intervals consisted of 250μg of HP in 50% saline and 50% Freunds Incomplete adjuvant (Merck 06-39-60-6) per mouse, intraperitoneally.

One week after the last injection the mice were bled by heart puncture and the serum tested for anti-peroxidase antibodies. The mouse showing the highest titred antiserum was selected for final boosting. This consisted of 200 μg of HP in 0,2 ml saline and was administered subcutaneously (0,1 ml) at two sites per mouse. The spleen was removed 3 days later.

The period between immunisations is important as too rapid successions of immunisations may result in lower antibody titre.

Serum was taken from the immunised mice not sacrificed. This was titred for anti-peroxidase antibody and kept in aliquots of 0,5 ml at -20° C for use in control assays.

1.2. Media

4

Standard Media

Dulbeccos Modified Eagles Medium (DMEM) (Flow Laboratories 1033120) Foetal Calf serum (FC) (Flow Laboratories) Sodium Pyruvate (NaPy) (Flow Laboratories 1682049) Penicillin 600 mg (Sodium Benzylpenicillin Glaxo) Streptomycin 1 gm (Glaxo) Sodium Bicarbonate (Merck 6329) Fungizone (Flow Laboratories 72436). This was made up according to the standard procedure as follows (Cold Spring Harbour manual on Hybridoma Techniques 1980), e.g.,to make up 10 litres of medium, one 10 litre pack of dry DMEM medium, 34g NaHCO₃, 1 ml fungizone, 600 mg of penicillin and 1 g of streptomycin were made up to 10 litres with distilled water. The pH was adjusted to 6.9 and media stored at 2 - 4° C. Foetal Calf serum and NaPy were added as required on use.

Polyethylene Glycol (PEG) Mol. Wt. 1500 (Merck 9727) was used as fusion promoter.

Selective Media

For preparation of the medium, hereinafter referred to as HAT selective medium, the following stock solution of hypoxanthine, aminopterin and thymidine (stock HAT solution) was used: hypoxanthine 5 000 $\mu$M, aminopterin 20 $\mu$M, thymidine 800 $\mu$M (Flow Laboratories 50 x stock 1680049). For preparation of the medium, hereinafter referred to as HT medium, the following stock solution of hypoxanthine and thymidine was used: hypoxanthine 5 000 $\mu$M, thymidine 800 $\mu$M (Flow Laboratories 50 x stock 1680949).

1.3. Cell Line : Sp2/O-Ag14 (Sp2) (Flow Laboratories 05-528-41).

This cell line is non-secreting and is derived from (x63-Ag8 x Balb/c) hybridoma.

1.4. Fusion Procedure

The fusion protocol is arranged in chronological order.

2 weeks prior to fusion : Sp2 cells thawed and cultured.

1 week prior to fusion : Sp2 cells tested for susceptibility to HAT selective medium.

3 days prior to fusion : Sp2 cells passed at 5 x 10⁴ in each of three 275 ml flasks (Costar 3275). Two of these flasks at ± 60% confluence yielded 5 x 10⁷ cells 3 days later. The remaining flask, as a back-up, could be cultured for re-freezing.

1 day prior to fusion : Feeder medium using spleen cells prepared according to the method described in the Cold Spring Harbour Hybridoma Manual (1980) with modifications, was as follows:

The spleen was removed aseptically from an 8-week-old Balb/c mouse killed by spinal dislocation. The spleen was placed in a petri dish (Nunc 150288) containing 15 ml DMEM at room temperature. In the hood it was teased with forceps, passed through a sieve and finally pipetted up and down to disrupt the cells further. The suspension was then transferred to a 50 ml polypropylene centrifuge tube and made up to 15 ml with DMEM and allowed to stand on ice for 10 minutes to allow clumps to settle and red blood cells to lyse. The supernatant was removed and washed twice with DMEM by centrifuging at 200 x g for 5 minutes each time. The cells were then re-suspended in 250 ml DMEM containing 10% FC, 1% NaPy and 5 ml of stock HAT solution, at a concentration of 1 - 2 x 10⁵/ml. 1 ml of this was pipetted into the wells of ten 24 well plates (Costar 3524) and incubated at 37° C with 7% CO₂.

Day of Fusion

Fusion method according to Köhler & Milstein was used with slight modifications. The spleen was removed aseptically from the selected immunised mouse and the same procedure was followed as in the preparation of the feeder layer (above). After the final wash the cells were re-suspended in 10 ml DMEM at a concentration of 1 - 2 x 10⁸ cells. These were mixed with a 10 ml suspension in DMEM of Sp2 cells at a concentration of 5 x 10⁷ cells which had been pipetted off the bottom of the flasks. The mixture was spun at 200 x g for seven minutes. After removing the supernatant the cells were fused using 50% PEG according to the standard procedure. They were eventually re-suspended in 250 ml DMEM with 10% FC, 1% NaPy and 5 ml of the stock HAT solution (final concentration 1 x HAT). 1 ml of this suspension was pipetted into the wells of the plates already containing the spleen feeder cells. Direct exposure to the selective HAT medium reduced labour. The plates were incubated as above.

3 Days After Fusion

All the media in the wells was aspirated and changed to DMEM containing 10% FC, 1% NaPy and 1 x HAT.

10 Days After Fusion

The medium was changed to HT medium (5 ml stock HT solution, 450 ml DMEN, 10% FC, 1% NaPy).

Hybrids were cultured and supernatants tested for production of antibody after 21 days from date of fusion.

1.5. Cloning Using Limiting Dilutions

Hybridoma cells in wells from which the supernatants revealed the presence of antibody were diluted at a ratio of 1 cell/0,1 ml and pipetted into 96 well cell-culture plates (Nunc 167008) containing a feeder medium of $10^5$ cells per 0,1 ml of spleen cells. The feeder cells were prepared the day before and incubated at 37°C in $CO_2$. All medium used for cloning was DMEM with 20% FC and 1% NaPy.

Those wells in which only one cell was visible under light microscopy were selected for subsequent testing for antibody. Limiting dilutions were continued (3 times) until all the single-cell clones yielded the required presence of antibody on testing.

1.6. Freezing

At each step the relevant cultures were frozen for later use.

2. Screening for Antibody

Assay for Antiperoxidase activity. Enzyme linked immunoabsorbant assay. (ELISA)

2.1. Chemicals and Equipment

Polystyrene 96 flat-bottomed welled plates (Nunc cat. No. 4-39454)

Horse Radish Peroxidase (Bayer - Miles 36-451-2, lot 8012 or 8042).

$Na_2CO_3$ (Merck, 6392)

$NaHCO_3$ (Merck, 6329)

$Na_2HPO_4 2H_2O$ (Merck, 6580)

$KH_2PO_4$ (Merck, 4873)

$KC\ell$ (SMM)

$NaC\ell$ (Saarchem, 582230)

Foetal Calf Serum (Flow Laboratories)

Tween 20 (Merck, 822184)

$\beta$-galactosidase-linked anti-mouse conjugate and Ortho-nitrophenyl-$\beta$-D-galactopyronaside (Amersham, N 831)

$MgC\ell_2 6H_2O$ (Koch Light Laboratories Ltd.)

2-mercaptoethanol (Merck, 805740)

Spectrophotometer (Unicam SP8-100)

2.2. Technique

The peroxidase solution was prepared by dissolving in deionised water and dialysing against 0.05 M Sodium Carbonate buffer, pH 9.5 at 4°C overnight. The plates were coated with the peroxidase by adding 100 $\mu$l of the peroxidase solution at 30 $\mu$g/ml to each well, (i.e. 3 $\mu$g peroxidase/well).

The plates were incubated for 2 hours at 37°C. They were then washed once with phosphate buffered saline (PBS), 0.01 M, pH 7.2. The residual binding sites were then blocked with 2% FCS in PBS by adding 300$\mu$l to each well and incubating for 1 hour at 37°C. The plates were then washed 3 times with PBS containing 0.1% v/v Tween 20. The coated plates were covered with paraffin film and stored at 4°C.

Test samples (supernatant or ascites fluid) were added to the wells. (100 $\mu$l per well) and incubated at 37°C for 1 hour. The wells were then washed 3 times with PBS-Tween.

100 $\mu$l of $\beta$-galactosidase-linked anti-mouse conjugate diluted in PBS containing 10mM $MgC\ell_2$, 0.1% v/v Tween 20 and 1mM 2-mercaptoethanol (0.8 $\mu$l conjugate per well) was added to each well. The plate was incubated at 37°C for 2 hours or overnight at 4°C, and then washed 3 times with PBS-Tween.

The substrate, ortho-nitrophenyl-$\beta$-D-galactopyranoside (ONPG) was then added, diluted in PBS containing 10mM $MgC\ell_2$, and 0.1M 2-mercaptoethanol (0.18 mg substrate per 150 $\mu$l per well) and the plate incubated at 37°C for 1 hour.

The reaction was stopped with 50$\mu$l of 1M $Na_2CO_3$. Although yellow colour indicates positive results quantitative analysis was done by diluting the contents of each well five times and reading the absorbance on a spectrophotometer at 410 nm. Relevant controls were used.

3. Purification of Monoclonal Antibodies

3.1. The monoclonal antibodies of the invention may be purified by standard methods of protein separation and purification described in the literature and known in the art, for example, by centrifugation after clarification of the ascites fluid by salt fractionation using a suitable salt, such as sodium sulphate or ammonium sulphate, by column chromatography on a suitable support medium including affinity column chromatography or by a combination of these methods. Two examples of suitable methods are described hereinunder.

### 3.2 Sodium and ammonium sulphate fractionation of anti-body containing ascites fluid

Ascites fluid was centrifuged at 12 000 x g for 30 minutes at 4°C. The clear supernatant was collected and an equal volume of 0.2 M phosphate buffer, pH 8.0 added. Solid $Na_2SO_4$ was added, slowly. With stirring at room temperature to give 18% $Na_2SO_4$.

The precipitate was allowed to form at room temperature for 1 hour. It was collected by centrifugation at 500xg for 10 minutes at room temperature. The pellet was dissolved in 0.1 M phosphate buffer, pH 8.0 in a volume equal to 80% of the original volume of ascites fluid used. Again, solid $Na_2SO_4$ was added, slowly, with stirring, at room temperature to give 18% $Na_2SO_4$. The precipitate was allowed to form and collected as above. The pellet was dissolved in 0.1 M phosphate buffer, pH 8.0 in a volume equal to 40% of the original volume of ascites fluid used. The solution was dialysed against 0.1 M phosphate buffer, pH 8.0 for 9 hours with 2 changes of buffer at 4°C. This solution containing purified anti-peroxidase was stored at -20°C.

In a similar manner ammonium sulphate may be used at 2 - 4°C at a concentration of 50% saturation with ammonium sulphate for the first and second precipitation.

### 3.3. Affinity Column Chromatography of antibody-containing ascites fluid

Materials Used

AH-Sepharose 4B (Pharmacia Fine Chemicals 170470-01)

Fluoro-dinitro-benzene (Merck 2966)

Absolute ethanol (Merck 983)

Sodium periodate (Merck 6597)

Ethylene glycol (Saarchem 223 300)

Sodium Borohydride (Merck 806372)

Sodium Acetate (Merck 6267)

Acetic Acid (Merck 63)

Formic Acid (Merck 264)

Peroxidase was conjugated to AH-Sepharose 4B using the periodate oxidation techniques as follows:

50 mg of peroxidase was dissolved in 10ml of freshly made 0.3 M sodium carbonate, pH 8.1. 1 ml of 1% fluorodinitrobenzene in absolute ethanol was added and mixed gently for 1 hour at room temperature. 10 ml of 0.06 M $NaIO_4$ in distilled water was added and mixed gently for 30 minutes at room temperature. 10 ml of 0.16 M ethylene glycol in distilled water was added and mixed gently for 1 hour at room temperature. The solution was then dialysed against about 1.5 litre of 0.01 M sodium carbonate buffer, pH 9.5 at 4°C. The buffer was changed 3 times over 24 hours.

An amount of 3.5 grams of AH-Sepharose 4B was washed several times with 0.5 M NaCl. The NaCl was then removed by washing with deionised water. The Sepharose was equilibrated with 0.01 M carbonate buffer, pH 9.5.

The equilibrated AH-Sepharose 4B was then added to the oxidised peroxidase solution. The mixture was stirred gently for 3 hours at room temperature. 50 mg of $NaBH_4$ was then added and left at 4°C overnight. The peroxidase conjugated Sepharose was washed alternately with 0.1 M acetate buffer, pH 4.0 containng 0.5 M NaCl, and 0.1 M phosphate buffer, pH 8.3 containing 0.5 M NaCl, four times each. The gel was then equilibrated with 0.15 M NaCl.

After the column (9 mm diameter, 8.5 cm high) was made, five ml of ascites fluid (previously centrifuged for 10 minutes) was applied to it. The column was washed with 0.15 M NaCl until no more protein was eluted. (2 ml fractions were collected and read at 280 nm).

The column was inverted and the adsorbed protein eluted using a step-wise pH gradient. The following four buffers were used:

0.01 M Acetate buffer, 0.5 M NaCl, pH 5.5

0.1 M Acetate buffer, 0.5 M NaCl, pH 4.0

0.1 M Acetate buffer, 0.5 M NaCl, pH 3.5

0.53 M Formate buffer, 0.15 M NaCl, pH 2.0

2 ml fractions were collected in tubes containing 600 $\mu l$ 1M Phosphate buffer, pH 8.0 to neutralise the eluates. The elution was monitored at 280 nm. The tubes of each protein peak were pooled and the protein concentration of each determined. The anti-peroxidase activity of the peaks was assayed using the ELISA technique.

### 4. Typing

Monoclonal culture supernatants and ascites fluid were typed using a mouse monoclonal typing kit, (Serotec, product code no : MMT 01K). This contained sheep anti-mouse immunoglobulin for testing the class and subclass specificity of mouse monoclonals. 75 $\mu$l of the test solution was pipetted into the central hole of one of the rosettes. The culture supernatants were used undiluted and the ascites fluid was diluted 1 in 100. 10 $\mu$l of each of the 6 antisera, $IgG_1$, $IgG_2a$, $IgG_2b$, $IgG_3$, IgA and IgM were pipetted into each of the outer 6 wells. The proteins were allowed to diffuse for 24 hours at room temperature. After this period, precipitin lines corresponding to the immunoglobulin specificity were observed.

The gel was then dried in contact with a piece of filter paper. The gel was stained with Kenacid Blue R for 30 minutes and then de-stained with water.

## 5. Purity of Antibody

The purity of the antibody of the invention and fractions containing it was monitored by standard methods known in the art such as sodium dodecyl sulphate-polyacrylamide gel electrophoresis (SDS - PAGE).

## 6. Results

10 days after fusion, hybrid clones were noted in all wells of the 10 plates. The supernatants were screened by ELISA (described above). Of these 18% showed the presence of antibodies to horse radish peroxidase. The results of the ELISA assay on the supernatants from these wells are shown in Figure 1. The cells from the well showing the highest concentration of antibody were selected for further cloning by limiting dilution.

The cells in the selected wells were subjected to cloning by limiting dilutions and the stable monoclonal producing cell line used for isolation, purification and characterisation of antibody. Each clone was subjected to 3 further limiting dilutions. Ascites fluids produced by the growth of the cloned cells in the abdominal cavity of Balb/c mice were assayed by the ELISA assay and the results are shown in Figure 2.

The ascites fluids from the cloned cell lines set out in Figure 2 were examined by a method described in paragraph 7 hereinafter for the presence of antibody capable of binding to horse radish peroxidase without significantly interfering with the catalytic activity of the enzyme to form a monoclonal peroxidase anti-peroxidase (PAP) complex. Of these fluids, only four were found to contain suitable antibody, i.e. antibodies of the class $IgG_1$ and having the characteristics identified above. These fluids are identified by the codes E1D, F11D, L5E and M6F in Figure 2. A number of the fluids were found to contain antibodies of the class $IgG_M$ which are unsuitable for the practice of the invention. Examples of such fluids are those identified by the codes G4F, G9G and J7F in Figure 2. Other fluids were found to contain antibodies of the class $IgG_1$ but not capable of producing PAP complex of the required characteristics. Examples of such fluids were those identified by the codes K4F and N5B in Figure 2.

## 7. Preparation of Monoclonal Peroxidase - Anti-peroxidase (PAP) Complexes

Monoclonal PAP complex was prepared using ammonium sulphate purified anti-peroxidase from ascites fluid in 0.05 M phosphate buffer, pH 7.5. To ensure a two to one ratio of peroxidase (PO) to anti-peroxidase (anti-PO), excess PO, typically a molar ratio of antibody to peroxidase of 3:1, (dialysed in the above buffer) was used.

The purified anti-PO was mixed with the PO solution and incubated for 2 hours at 37°C. The PAP complex was separated from the excess PO by gel filtration on Sephacryl S-200 (Pharmacia) (2,6 x 85 cm), equilibrated with the above buffer. Three ml fractions were collected at room temperature at 20 ml/hr. The absorbance of each fraction was read at 280 nm and 403 nm on a Unicam SP8-100 spectrophotometer.

The PAP complex fractions were pooled and the RZ value (O.D. 403 nm/O.D. 280 nm) and protein concentration measured. The PAP solution was assayed for PO activity in the following way. The solution was diluted to about 1$\mu$g protein in 3 ml 0,1 phosphate buffer, pH 7,0. The temperature was maintained at 25°C. 50 $\mu$l of an aqueous solution of guaiacol (0.02 M) was added. After adding 30 $\mu$l of an aqueous solution of hydrogen peroxide (12,3 mM), the solution was mixed and the absorbance read at 436 nm after exactly 1 minute. The PO activity was read off a standard curve run at the same time as the unknown sample.

The molecular ratio (MR) of PO to anti-PO in PAP was calculated as follows:

$$\text{conc. of PO in PAP} \quad = \quad \frac{\text{OD}_{403} \text{ of PAP}}{\text{OD}_{403} \text{ of PO (at 1 mg/ml)}} \quad \text{mg/ml}$$

$$\text{OD}_{280} \text{ of PO in PAP} \quad = \quad \frac{\text{OD}_{403} \text{ of PAP}}{\text{RZ of PO}}$$

$$\text{OD}_{280} \text{ of anti-PO in PAP} \quad = \quad \text{OD}_{280} \text{ of PAP} - \text{OD}_{280} \text{ of PO in PAP}$$

$$\text{Conc. of anti-PO in PAP} \quad = \quad \frac{\text{OD}_{280} \text{ of anti-PO in PAP}}{\text{OD}_{280} \text{ of anti-PO (at 1 mg/ml)}} \quad \text{mg/ml}$$

$$\text{MR of PO to anti-PO in PAP} = \quad \frac{\text{conc. of PO}}{\text{conc. of anti-PO}} \quad \times \ 4$$

The PAP complex solutions were stored at 4°C in the presence of 2% bovine serum albumin (BSA) (Sigma A-4378) and 0.01% Thimerosal (Merck 818957).

Of the various ascites fluids set out in Figure 2, only four monoclonal antibodies purified therefrom as already described produced PAP having the required characteristics, in particular the molar ratios of peroxidase to antibody of at least 1,78 and an RZ value of at least 0,55. The molecular weight of these PAP complexes was determined by elution volume on gel filtration using Sephacryl S-200 and was found to be about 240,000. The results of this analysis are shown graphically by Figure 3. Clearly therefore these monoclonal PAP complexes are different to the PAP complexes produced by Mason et al as hereinbefore described. Furthermore, in all complexes the enzyme activity had been reduced by less than 30%. The results obtained for these four PAP complexes are set out in Table I below:

## T A B L E   I

| Cell Line | Sub-Class | RZ | Molar Ratio | U perox/mg |
|-----------|-----------|------|------------|------------|
| E1D | $IgG_1$ | 0.55 | 1.78 | 67.5 |
| F11D | $IgG_1$ | 0.55 | 1.70 | 59.3 |
| L5E | $IgG_1$ | 0.57 | 1.59 | 62.8 |
| M6F | $IgG_1$ | 0.59 | 1.96 | 67.1 |

Production of ascites fluid from cell lines E1D, F11D, L5E and M6F was also examined. The mice used were all Balb/c mice from the same genetic stock. The mice were 8 weeks old at the time of the first injection of cells and all mice had been pristane-primed 10 days prior to injection 3 taps were taken. The interval between the taps was an average of 2 days. The results obtained are set out in Table II below:

## T A B L E   II

| Cell Line | First Tap Average ml/mouse | Second Tap Average ml/mouse | Third Tap Average ml/mouse | Total Volume ml/mouse |
|-----------|----------------------------|------------------------------|-----------------------------|------------------------|
| E1D | 2,5 | 1,6 | 1,6 | 5,7 |
| F11D | 2,9 | 1,5 | 1,4 | 5,8 |
| L5E | 3,0 | 2,0 | 1,8 | 6,8 |
| M6F | 3,5 | 3,4 | 2,7 | 9,6 |

8. Confirmation of the Production of Monoclonal Antibody against Horse Radish Peroxidase by Hybrid Cell Line M6F (C5G12F)

8.1. Materials
Diaminobenzidine (BDH, 13033)
$H_2O_2$ 30% (Merck, 7210)
Glacial acetic acid (Merck, 63)
Sodium veranol (Merck, 27283)
Glycerol

8.2. Procedure
Hybrid cells were treated with horse radish peroxidase, washed and stained with diaminobenzidine to detect antibody to the peroxidase:
8.2.1. Cells were cytocentrifuged onto clean slides,
8.2.2. Air dried and fixed in ethanol and 1% glacial acetic acid for three minutes,
8.2.3. Washed in TBS (0,15 M sodium chloride containing 0.05 M Tris HCl pH 7.6),
8.2.4. Incubated at room temperature (and some at 37°C) with 0 5 mg/ml horse radish peroxidase. (5 mg in 10 ml TBS),
8.2.5. Washed in TBS and treated with DAB. 5 mg in 10 ml TBS filtered and centrifuged for 5 minutes at 1 000 rpm. 3 drops of 30% $H_2O_2$ was added. Incubated at room temperature for up to 1 hour.
8.2.6. Washed in TBS.

EP 0 137 657 B1

8.2.7. 0.5 g sodium veranol and 0.31 g NaCℓ in 50 ml distilled water was adjusted to pH.8 6 with 1.0N HCℓ. Equal volumes of this solution and glycerol were mixed and the resulting mixture used for mounting.

8.2.8. Sp2/O-Ag14 cells were treated in a similar manner as controls.

8.3. Results

The hybridoma cells showed brown staining over the surface of the cells whilst no brown staining was observed in the control.

**Claims**

1.  A mouse monoclonal anti-peroxidase antibody characterised in that it is:
    (a) of the sub-class $IgG_1$;
    (b) capable of binding to horse radish peroxidase to form a complex without reducing the catalytic activity of the enzyme by more than 30% the complex having a molar ratio of peroxidase to antibody of at least 1.78:1 and having an RZ value of at least 0.55.

2.  A monoclonal antibody as claimed in claim 1 produced by a hybridoma formed by fusion of spleen cells from a Balb/c mouse previously immunised with horse radish peroxidase and cells from the mouse myeloma cell line Sp2/O-Ag14.

3.  A monoclonal antibody as claimed in claim 1 or claim 2 capable of binding to horse radish peroxidase to form a complex containing a molar ratio of peroxidase to antibody of at least 1.96:1.

4.  A monoclonal antibody as claimed in any one of claims 1 to 3 capable of binding to horse radish peroxidase to form a complex with an RZ value of at least 0.59.

5.  A hybridoma formed by fusion of spleen cells from a Balb/c mouse previously immunised with horse radish peroxidase and cells from a Sp2/O-Ag14 mouse myeloma cell line, said hybridoma being capable of producing a monoclonal antibody as claimed in any one of the preceding claims.

6.  A method of producing a hybridoma as claimed in claim 5 including the steps of:
    (i) immunising a Balb/c mouse with horse radish peroxidase enzyme;
    (ii) removing the spleen from the mouse and extracting the spleen cells;
    (iii) fusing the spleen cells with Sp2/O-Ag14 mouse myeloma cells in the presence of a cell fusion promotor;
    (iv) diluting and culturing the fused cells in containers in a medium which will not support the unfused myeloma cells;
    (v) examining the supernatant in each container containing hybridomas for the presence of the desired antibody; and
    (vi) selecting and cloning the hybridomas which produce the desired antibody.

7.  A method as claimed in claim 6 wherein the extraction of spleen cells in step (ii) is achieved by making a suspension of the spleen cells.

8.  A method as claimed in claim 6 or 7 wherein the cell fusion promoter employed in step (iii) is polyethylene glycol.

9.  A method as claimed in claims 6 to 8 wherein step (iv) is carried out in the presence of feeder spleen cells.

10. A method as claimed in claims 6 to 9 wherein the medium used in step (iv) contains appropriate concentrations of hypoxanthine, aminopterin and thymidine.

11. A method as claimed in any one of claims 6 to 10 wherein cloning in step (vi) is achieved by the technique of limiting dilution.

12. A method as claimed in any one of claims 6 to 11 including the steps of:

(i) immunising a Balb/c mouse with horse radish peroxidase enzyme by injecting subcutaneously 10-2000 µg of the enzyme in an adjuvant medium, followed by subsequent injections at spaced intervals, e.g. three injections at about four-weekly intervals, each of 10-2000 µg of the enzyme in an adjuvant medium, followed by a final booster dose of 10-500 µg of the enzyme in saline administered subcutaneously;

(ii) removing the spleen aseptically from the mouse and making a suspension of spleen cells;

(iii) fusing the spleen cells with Sp2/O-Ag14 mouse myeloma cells in the presence of a cell fusion promoter;

(iv) diluting and culturing the fused cells in suitable containers in a medium which will not support the growth of unfused myeloma cells in the presence of feeder spleen cells prepared from the spleen of a young mouse;

(v) examining the supernatant in each container containing hybridomas for the presence of the desired antibody; and

(vi) selecting and cloning by the technique of limiting dilution, the hybridomas which produce the desired antibody.

13. A method for the preparation of a mouse monoclonal anti-peroxidase antibody as claimed in any of claims 1 to 4 which comprises culturing a hybridoma as defined in claim 7, if desired in vivo , and isolating said antibody.

14. A complex containing a molar ratio of horse radish peroxidase to mouse monoclonal anti-peroxidase antibody of at least 1.78:1 and with an RZ value of at least 0.55, the antibody used being an antibody as claimed in any one of claims 1 to 4.

15. A complex as claimed in claim 14 containing a molar ratio of peroxidase to mouse monoclonal anti-peroxidase antibody of at least 1.96:1.

16. A complex as claimed in claim 14 or 15 with an RZ value of at least 0.59.

Claims for the following Contracting State: AT

1. A method of preparing a hybridoma which produces a mouse monoclonal anti-peroxidase antibody with the following characteristics:

(a) of the sub-class IgG$_1$;

(b) capable of binding to horse radish peroxidase to form a complex without reducing the catalytic activity of the enzyme by more than 30%, the complex having a molar ratio of peroxidase to antibody of at least 1.78:l and having an RZ value of at least 0.55,

which comprises

(i) immunising a Balb/c mouse with horse radish peroxidase enzyme;

(ii) removing the spleen from the mouse and extracting the spleen cells;

(iii) fusing the spleen cells with Sp2/O-Ag14 mouse myeloma cells in the presence of a cell fusion promotor;

(iv) diluting and culturing the fused cells in containers in a medium which will not support the unfused myeloma cells;

(v) examining the supernatant in each container containing hybridomas for the presence of the desired antibody; and

(vi) selecting and cloning the hybridomas which produce the desired antibody.

2. A method as claimed in claim 1 wherein the extraction of spleen cells in step (ii) is achieved by making a suspension of the spleen cells.

3. A method as claimed in claim 1 or 2 wherein the cell fusion promotor employed in step (iii) is polyethylene glycol.

4. A method as claimed in claims 1 to 3 wherein step (iv) is carried out in the presence of feeder spleen cells.

12

EP 0 137 657 B1

5. A method as claimed in claims 1 to 4 wherein the medium use in step (iv) contains appropriate concentrations of hypoxanthine, aminopterin and thymidine.

6. A method as claimed in any one of claims 1 to 5 wherein cloning in step (vi) is achieved by the technique of limiting dilution.

7. A method as claimed in any one of claims 1 to 6 including the steps of:
(i) immunising a Balb/c mouse with horse radish peroxidase enzyme by injecting subcutaneously 10-2000 $\mu g$ of the enzyme in an adjuvant medium, followed by subsequent injections at spaced intervals, e.g. three injections at about four-weekly intervals, each of 10-2000 $\mu g$ of the enzyme in an adjuvant medium, followed by a final booster dose of 10-500 $\mu g$ of the enzyme in saline administered subcutaneously;
(ii) removing the spleen aseptically from the mouse and making a suspension of spleen cells;
(iii) fusing the spleen cells with Sp2/O-Ag14 mouse myeloma cells in the presence of a cell fusion promoter;
(iv) diluting and culturing the fused cells in suitable containers in a medium which will not support the growth of unfused myeloma cells in the presence of feeder spleen cells prepared from the spleen of a young mouse;
(v) examining the supernatant in each container containing hybridomas for the presence of the desired antibody; and
(vi) selecting and cloning by the technique of limiting dilution, the hybridomas which produce the desired antibody.

8. A method for the preparation of a mouse monoclonal anti-peroxidase antibody with the following characteristics:
(a) of the sub-class $IgG_1$;
(b) capable of binding to horse radish peroxidase to form a complex without reducing the catalytic activity of the enzyme by more than 30%, the complex containing a molar ratio of peroxidase to antibody of at least 1.78:1 and having an RZ value of at least 0.55, which comprises culturing a hybridoma, prepared as claimed in any of claims 1 to 7, if desired in vivo, and isolating said antibody.

**Revendications**

1. Un anticorps monoclonal de souris anti-peroxydase caractérisé en ce que :
(a) il appartient à la sous-classe $IgG_1$ ;
(b) il est capable de se lier à la peroxydase de raifort pour former un complexe, sans réduire l'activité catalytique de l'enzyme de plus de 30 %, le complexe ayant un rapport molaire de la peroxydase à l'anticorps d'au moins 1,78/1 et ayant une valeur RZ d'au moins 0,55.

2. Un anticorps monoclonal selon la revendication 1, produit par un hybridome formé par fusion de cellules spléniques d'une souris Balb/c préalablement immunisée avec de la peroxydase de raifort et de cellules de la lignée de cellules de myélome de souris Sp2/O-Ag14.

3. Un anticorps monoclonal selon la revendication 1 ou la revendication 2 capable de se fixer à la peroxydase de raifort pour former un complexe contenant un rapport molaire de la peroxydase à l'anticorps d'au moins 1,96/1.

4. Un anticorps monoclonal selon l'une quelconque des revendications 1 à 3 capable de se fixer à la peroxydase de raifort pour former un complexe ayant une valeur RZ d'au moins 0,59.

5. Un hybridome formé par fusion de cellules spléniques d'une souris Balb/c préalablement immunisée avec de la peroxydase de raifort et de cellules d'une lignée de cellules de myélome de souris Sp2/O-Ag14, ledit hybridome étant capable de produire un anticorps monoclonal selon l'une quelconque des revendications précédentes.

6. Un procédé pour produire un hybridome selon la revendication 5 comprenant les étapes de :

13

EP 0 137 657 B1

(i) immunisation d'une souris Balb/c avec l'enzyme peroxydase de raifort ;
(ii) prélèvement de la rate de la souris et extraction des cellules spléniques ;
(iii) fusion des cellules spléniques avec des cellules de myélome de souris Sp2/O-Ag14 en présence d'un promoteur de fusion cellulaire ;
(iv) dilution et culture des cellules fusionnées dans des récipients dans un milieu qui n'entretient pas les cellules de myélome non fusionnées ;
(v) examen du surnageant dans chaque récipient contenant des hybridomes pour déterminer la présence de l'anticorps désiré ; et
(vi) sélection et clonage des hybridomes produisant l'anticorps désiré.

7. Un procédé selon la revendication 6, dans lequel l'extraction des cellules spléniques dans l'étape (ii) est effectuée par formation d'une suspension des cellules spléniques.

8. Un procédé selon la revendication 6 ou 7, dans lequel le promoteur de fusion cellulaire utilisé dans l'étape (iii) est le polyéthylèneglycol.

9. Un procédé selon les revendications 6 à 8, dans lequel l'étape (iv) est effectuée en présence de cellules spléniques nourricières.

10. Un procédé selon les revendications 6 à 9, dans lequel le milieu utilisé dans l'étape (iv) contient des concentrations appropriées d'hypoxanthine, d'aminoptérine et de thymidine.

11. Un procédé selon l'une quelconque des revendications 6 à 10, dans lequel le clonage dans l'étape (vi) est effectué selon la technique de la dilution limitante.

12. Un procédé selon l'une quelconque des revendications 6 à 11, comprenant les étapes de :
(i) immunisation d'une souris Balb/c avec l'enzyme peroxydase de raifort par injection sous-cutanée de 10 à 2 000 $\mu$g de l'enzyme dans un milieu adjuvant, suivie d'injections ultérieures à des intervalles espacés, par exemple trois injections à des intervalles d'environ 4 semaines, chacune de 10 à 2 000 $\mu$g de l'enzyme dans un milieu adjuvant, suivies d'une dose finale de rappel de 10 à 500 $\mu$g de l'enzyme dans du soluté salé administrée par voie sous-cutanée ;
(ii) prélèvement aseptique de la rate de la souris et formation d'une suspension de cellules spléniques ;
(iii) fusion des cellules spléniques avec des cellules de myélome de souris Sp2/O-Ag14 en présence d'un promoteur de fusion cellulaire ;
(iv) dilution et culture des cellules fusionnées dans des récipients appropriés dans un milieu qui n'entretient pas la croissance des cellules de myélome non fusionnées en présence de cellules spléniques nourricières préparées à partir de la rate d'une jeune souris ;
(v) examen du surnageant de chaque récipient contenant des hybridomes pour déterminer la présence de l'anticorps désiré ; et
(vi) sélection et clonage, selon la technique de la dilution limitante, des hybridomes qui produisent l'anticorps désiré.

13. Un procédé pour la préparation d'un anticorps monoclonal de souris anti-peroxydase selon l'une quelconque des revendications 1 à 4, qui comprend la culture d'un hybridome comme défini dans la revendication 7, si on le désire in vivo, et l'isolement dudit anticorps.

14. Un complexe contenant un rapport molaire de la peroxydase de raifort à un anticorps monoclonal de souris anti-peroxydase d'au moins 1,78/1 et ayant une valeur RZ d'au moins 0,55, l'anticorps utilisé étant un anticorps selon l'une quelconque des revendications 1 à 4.

15. Un complexe selon la revendication 14 contenant un rapport molaire de la peroxydase à l'anticorps monoclonal de souris anti-peroxydase d'au moins 1,96/1.

16. Un complexe selon la revendication 14 ou 15 ayant une valeur RZ d'au moins 0,59.

Revendications pour l'Etat contractant suivant: AT

14

1. Un procédé pour la préparation d'un hybridome produisant un anticorps monoclonal de souris anti-peroxydase caractérisé en ce que :

(a) il appartient à la sous-classe IgG$_1$;

(b) il est capable de se lier à la peroxydase de raifort pour former un complexe, sans réduire l'activité catalytique de l'enzyme de plus de 30%, le complexe ayant un rapport molaire de la peroxydase á l'anticorps d'au moins 1,78/1 et ayant une valuer RZ d'au moins 0,55,

ce procédé comprenant les étapes de :

(i) immunisation d'une souris Balb/c avec l'enzyme peroxydase de raifort;

(ii) prélèvement de la rate de la souris et extraction des cellules spléniques;

(iii) fusion des cellules spléniques avec des cellules de myélome de souris Sp2/O-Ag14 en présence d'un promoteur de fusion cellulaire;

(iv) dilution et culture des cellules fusionnées dans des récipients dans un milieu qui n'entretient pas les cellules de myélome non fusionnées;

(v) examen du surnageant dans chaque récipient contenant des hybridomes pour déterminer la présence de l'anticorps désiré; et

(vi) sélection et clonage des hybridomes produisant l'anticorps désiré.

2. Un procédé selon la revendication 1, dans lequel l'extraction des cellules spléniques dans l'etape (ii) est effectuée par formation d'une suspension des cellules spléniques.

3. Un procédé selon les rèvendication 1 ou 2, dans lequel le promoteur de fusion cellulaire utilisé dans l'étape (iii) est le polyéthylèneglycol.

4. Un procédé selon les revendications 1 à 3, dans lequel l'étape (iv) est effectuée en présence de cellules spléniques nourricières.

5. Un procédé selon les revendications 1 à 4, dans lequel le milieu utilisé dans l'étape (iv) contient des concentrations appropriées d'hypoxanthine, d'aminoptérine et de thymidine.

6. Un procédé selon l'une quelconque des revendications 1 à 5, dans lequel le clonage dans l'etape (vi) est effectué selon la technique de la dilution limitante.

7. Un procédé selon l'une quelconque des revendications 1 à 6, comprenant les étapes de:

(i) immunisation d'une souris Balb/c avec l'enzyme peroxydase de raifort par injection sous-cutanée de 10 à 2000 μg de l'enzyme dans un milieu adjuvant, suivie d'injections ultérieures à des intervalles espacés, par exemple trois injections à des intervalles d'environ 4 semaines, chacune de 10 à 2000 μg de l'enzyme dans un milieu adjuvant, suivies d'une dose finale de rappel de 10 à 500 μg de l'enzyme dans du soluté salé administrée par voie sous-cutanée;

(ii) prélèvement aseptique de la rate de la souris et formation d'une suspension de cellules spléniques; 1

(iii) fusion des cellules spléniques avec des cellules de myélome de souris Sp2/O-Ag14 en présence d'un promoteur de fusion cellulaire;

(iv) dilution et culture des cellules fusionnées dans des récipients appropriés dans un milieu qui n'entretient pas la croissance des cellules de myélome non fusionnées en présence de cellules spléniques nourriciéres préparées à partir de la rate d'une jeune souris;

(v) examen du surnageant de chaque récipient contenant des hybridomes pour déterminer la présence de l'anticorps désiré; et

(vi) sélection et clonage, selon la technique de la dilution limitante, des hybridomes qui produisent l'anticorps désiré.

8. Un procédé pour la préparation d'un anticorps monoclonal de souris anti-peroxydase caractérisé en ce que:

(a) il appartient à la sous-classe IgG$_1$;

(b) il est capable de se lier à la peroxydase de raifort pour former un complexe, sans réduire l'activité catalytique de l'enzyme de plus de 30%, le complexe ayant un rapport molaire de la peroxydase á l'anticorps d'au moins 1,78/1 et ayant une valuer RZ d'au moins 0,55, le procédé comprenant la culture d'un hybridome préparé selon l'une quelconque des revendications 1 à 7, si on le désire in vivo , et l'isolement dudit anticorps.

**Ansprüche**

1. Monoklonaler Maus-Anti-Peroxidase-Antikörper, dadurch gekennzeichnet, daß er
   (a) der Unterklasse IgG$_1$ angehört;
   (b) zur Bindung an Meerrettich-Peroxidase zur Bildung eines Komplexes in der Lage ist, ohne die katalytische Aktivität des Enzymes um mehr als 30 % zu verringern, wobei der Komplex ein molares Verhältnis von Peroxidase zu Antikörper von mindestens 1,78:1 und einen RZ-Wert von mindestens 0,55 hat.

2. Monoklonaler Antikörper nach Anspruch 1, erzeugt von einem Hybridom, das durch Fusion von Milzzellen aus einer Balb/c-Maus, die zuvor mit Meerrettich-Peroxidase immunisiert worden ist, mit Zellen von der Maus-Myelom-Zellinie Sp2/O-Ag14 gebildet worden ist.

3. Monoklonaler Antikörper nach einem der Ansprüche 1 oder 2, der zur Bindung an Meerrettich-Peroxidase zur Bildung eines Komplexes, enthaltend ein molares Verhältnis von Peroxidase zu Antikörper von mindestens 1,96:1, fähig ist.

4. Monoklonaler Antikörper nach einem der Ansprüche 1 bis 3, der zur Bindung an Meerrettich-Peroxidase zur Bildung eines Komplexes mit einem RZ-Wert von mindestens 0,59 fähig ist.

5. Hybridom, gebildet durch Fusion von Milzzellen aus einer Balb/c-Maus, die zuvor mit Meerrettich-Peroxidase immunisiert worden ist, und Zellen aus einer Sp2/O-Ag14 Maus-Myelom-Zellinie, wobei das Hybridom zur Erzeugung eines monoklonalen Antikörpers nach einem der vorstehenden Ansprüche fähig ist.

6. Verfahren zum Erzeugen eines Hybridoms nach Anspruch 5, umfassend die folgenden Schritte:
   (i) Immunisieren einer Balb/c-Maus mit dem Enzym Meerrettich-Peroxidase;
   (ii) Entfernen der Milz aus der Maus und Extraktion der Milzzellen;
   (iii) Fusion der Milzzellen mit Sp2/O-Ag14 Maus-Myelom-Zellen in Gegenwart eines Zellfusionspromotors;
   (iv) Verdünnen und Kultivieren der fusionierten Zellen in Behältnissen in einem Medium, das das Wachstum von nicht fusionierten Myelom-Zellen nicht fördert;
   (v) Untersuchen des Überstandes aus jedem Hybridome enthaltenden Behältnis auf die Gegenwart des gewünschten Antikörpers; und
   (vi) Auswählen und Klonieren der den gewünschten Antikörper erzeugenden Hybridome.

7. Verfahren nach Anspruch 6, wobei die Extraktion von Milzzellen in Schritt (ii) durch Herstellen einer Suspension der Milzzellen erreicht wird.

8. Verfahren nach einem der Ansprüche 6 oder 7, wobei der Zellfusionspromotor, der in Schritt (iii) eingesetzt wird, Polyethylenglycol ist.

9. Verfahren nach einem der Ansprüche 6 bis 8, wobei der Schritt (iv) in Gegenwart von Nähr-Milzzellen durchgeführt wird.

10. Verfahren nach einem der Ansprüche 6 bis 9, wobei das in Schritt (iv) verwendete Medium eine geeignete Konzentration von Hypoxanthin, Aminopterin und Thymidin enthält.

11. Verfahren nach einem der Ansprüche 6 bis 10, wobei das Klonieren in Schritt (vi) durch das Verfahren der limitierenden Verdünnung erreicht wird.

12. Verfahren nach einem der Ansprüche 6 bis 11 einschließlich der folgenden Schritte:
    (i) Immunisieren einer Balb/c-Maus mit Meerrettich-Peroxidase-Enzym durch subkutane Injektion von 10 - 2000 μg des Enzymes in einem Adjuvanz-Medium, gefolgt von daran anschließenden Injektionen in zeitlichen Abständen, beispielsweise drei Injektionen von jeweils 10 - 2000 μg des Enzymes in einem Adjuvanz-Medium in ungefähr vierwöchigen Abständen, gefolgt von einer abschließenden Booster-Dosis von 10 - 500 μg des Enzymes in Kochsalzlösung, die subkutan verabreicht wird;
    (ii) aseptisches Entfernen der Milz aus der Maus und Herstellen einer Suspension von Milzzellen;

16

EP 0 137 657 B1

(iii) Fusionieren der Milzzellen mit Sp2/O-Ag14 Maus-Myelom-Zellen in Gegenwart eines Zellfusionspromotors;

(iv) Verdünnen und Kultivieren der fusionierten Zellen in geeigneten Behältnissen in einem Medium, das das Wachstum nicht fusionierter Myelom-Zellen nicht fördert, in Gegenwart von Nähr-Milzzellen, die aus der Milz einer jungen Maus gewonnen worden sind;

(v) Untersuchen des Überstandes in jedem Hybridome enthaltenden Behältnis auf die Gegenwart des gewünschten Antikörpers; und

(vi) Auswählen und Klonieren der die gewünschten Antikörper erzeugenden Hybridome, durch das Verfahren der limitierenden Verdünnung.

13. Verfahren zum Herstellen eines monoklonalen Maus-Anti-Peroxidase-Antikörpers nach einem der Ansprüche 1 bis 4, das das Kultivieren eines wie in Anspruch 7 definierten Hybridoms, wenn gewünscht in vivo , und das Isolieren des Antikörpers umfaßt.

14. Komplex, enthaltend ein molares Verhältnis von Meerrettich-Peroxidase zu monoklonalen Maus-Anti-Peroxidase-Antikörper von mindestens 1,78:1, mit einem RZ-Wert von mindestens 0,55, wobei der verwendete Antikörper ein Antikörper nach einem der Ansprüche 1 bis 4 ist.

15. Komplex nach Anspruch 14, enthaltend ein molares Verhältnis von Peroxidase zu monoklonalem Maus-Anti-Peroxidase-Antikörper von mindestens 1,96:1.

16. Komplex nach einem der Ansprüche 14 oder 15 mit einem RZ-Wert von mindestens 0,59.

Patentansprüche für folgenden Vertragsstaat: AT

1. Verfahren zum Herstellen eines Hybridoms, das einen monoklonalen Maus-Anti-Peroxidase-Antikörper erzeugt, dadurch gekennzeichnet, daß er

(a) der Unterklasse IgG$_1$ angehört;

(b) zur Bindung an Meerrettich-Peroxidase zur Bildung eines Komplexes in der Lage ist, ohne die katalytische Aktivität des Enzymes um mehr als 30% zu verringern, wobei der Komplex ein molares Verhältnis von Peroxidase zu Antikörper von mindestens 1,78:1 und einen RZ-Wert von mindestens 0,55 hat.

Das Verfahren umfaßt

(i) Immunisieren einer Balb/c-Maus mit dem Enzym Meerrettich-Peroxidase;

(ii) Entfernen der Milz aus der Maus und Extraktion der Milzzellen;

(iii) Fusion der Milzzellen mit Sp2/-O-Ag14 Maus-Myelom-Zellen in Gegenwart eines Zellfusionspromotors;

(iv) Verdünnen und Kultivieren der fusionierten Zellen in Behältnissen in einem Medium, das das Wachstum von nicht fusionierten Myelom-Zellen nicht fördert;

(v) Untersuchen des Überstandes aus jedem Hybridome enthaltenden Behältnis auf die Gegenwart des gewünschten Antikörpers; und

(vi) Auswählen und Klonieren der den gewünschten Antikörper erzeugenden Hybridome.

2. Verfahren nach Anspruch 1, wobei die Extraktion von Milzzellen in Schritt (ii) durch Herstellen einer Suspension der Milzzellen erreicht wird.

3. Verfahren nach einem der Ansprüche 1 oder 2, wobei der Zellfusionspromotor, der in Schritt (iii) eingesetzt wird, Polyethylenglycol ist.

4. Verfahren nach einem der Ansprüche 1 bis 3, wobei der Schritt (iv) in Gegenwart von Nähr-Milzzellen durchgeführt wird.

5. Verfahren nach einem der Ansprüche 1 bis 4, wobei das in Schritt (iv) verwendete Medium eine geeignete Konzentration von Hypoxanthin, Aminopterin und Thymidin enthält.

6. Verfahren nach einem der Ansprüche 1 bis 5, wobei das Klonieren in Schritt (vi) durch das Verfahren der limitierenden Verdünnung erreicht wird.

17

7. Verfahren nach einem der Ansprüche 1 bis 6 einschließlich der folgenden Schritte:

(i) Immunisieren einer Balb/c-Maus mit Meerrettich-Peroxidase-Enzym durch subkutane Injektion von 10 - 2000 μg des Enzymes in einem Adjuvanz-Medium, gefolgt von daran anschließenden Injektionen in zeitlichen Abständen, beispielsweise drei Injektionen von jeweils 10 - 2000 μg des Enzymes in einem Adjuvanz-Medium in ungefähr vierwöchigen Abständen, gefolgt von einer abschließenden Booster-Dosis von 10 - 500 μg des Enzymes in Kochsalzlösung, die subkutan verabreicht wird;

(ii) aseptisches Entfernen der Milz aus der Maus und Herstellen einer Suspension von Milzzellen;

(iii) Fusionieren der Milzzellen mit Sp2/O-Ag14 Maus-Myelom-Zellen in Gegenwart eines Zellfusionspromotors;

(iv) Verdünnen und Kultivieren der fusionierten Zellen in geeigneten Behältnissen in einem Medium, das das Wachstum nicht fusionierter Myelom-Zellen nicht fördert, in Gegenwart von Nähr-Milzzellen, die aus der Milz einer jungen Maus gewonnen worden sind;

(v) Untersuchen des Überstandes in jedem Hybridome enthaltenden Behältnis auf die Gegenwart des gewünschten Antikörpers; und

(vi) Auswählen und Klonieren der die gewüschten Antikörper erzeugenden Hybridome, durch das Verfahren der limitierenden Verdünnung.

8. Verfahren zum Herstellen eines monoklonalen Maus-Anti-Peroxidase-Antikörpers, dadurch gekennzeichnet, daß er

(a) der Unterklasse IgG$_1$ angehört;

(b) zur Bindung an Meerrettich-Peroxidase zur Bildung eines Komplexes in der Lage ist, ohne die katalytische Aktivität des Enzymes um mehr als 30% zu verringern, wobei der Komplex ein molares Verhältnis von Peroxidase zu Antikörper von mindestens 1,78:1 und einen RZ-Wert von mindestens 0,55 hat. Das Verfahren umfaßt das Kultivieren eines nach einem der Ansprüche 1 bis 7 erzeugten Hybridoms, wenn gewünscht in vivo , und das Isolieren des Antikörpers.

Fig.1.

Fig.2.

## *Fig.3.*

GEL FILTRATION USING SEPHACRYL−S 200